# DEMANDE DE BREVET EUROPEEN

(11) **EP 1 595 512 A1**
(43) Date de publication de la demande: **16.11.2005**
(21) Numéro de dépôt: 05076084.2
(22) Date de dépôt: 09.05.2005
(51) Int. Cl.: A61F 2/00

(54) **Prothese de réfection anatomique**

(30) Priorité: 14.05.2004 FR 0405253
(71) Demandeur: Urogyn, 34000 Montpellier (FR)
(72) Inventeur: Decoux, Jean-Jacques, 34070 Montpellier (FR); Dieudonne, Gabriel, 06800 Cagnes sur Mer (FR); Fonfreide, Gérard, 34130 Mauguio (FR); Toledo, Ludovic, 34000 Montpellier (FR)
(74) Mandataire: Junca, Eric

(57) **Abrégé**

Cette plaque de réfection anatomique, destinée à la réparation de hernies et particulièrement mais non exhaustivement de hernies inguinales et crurales, est caractérisée en ce qu'elle est constituée d'une poche (1) de forme sensiblement pyramidale tronquée ou parallélépipédique, et associée par l'une de ses faces (2) à une collerette (3) munie d'au moins un orifice (4).

## Description

La présente invention a pour objet une prothèse de réfection anatomique destinée particulièrement, mais non limitativement, à la réparation des hernies inguinales, crurales et ombilicales.

De telles affections proviennent d'une faiblesse de la paroi abdominale. Cette paroi abdominale est constituée d'éléments musculaires, tendineux, ligamentaires et graisseux, et est tapissée par une membrane séreuse (le péritoine) contenant les viscères. Dans le cas de hernies, cette paroi s'invagine et forme un sac dans lequel peut s'engager le péritoine et éventuellement des viscères prolabées. Dans certains cas de hernies, un cordon anatomique, contenant par exemple des vaisseaux et des nerfs, se situe au contact direct de la hernie. Dans certains autres cas, le sac herniaire longe ce cordon.

De manière générale, les hernies peuvent être traitées chirurgicalement soit sans implantation de prothèse soit avec implantation de prothèse.

Dans le premier cas, il s'agit de refermer l'orifice herniaire en utilisant les tissus sains environnants et en les associant par suture, agrafe ou collage. Ces tissus, étant mis en tension, peuvent se dissocier sous l'effet de contraintes physiologiques, provoquant ainsi une récidive de hernie.

Dans le deuxième cas, il s'agit de compenser les faiblesses pariétales par l'ajout d'un tissu prothétique, ou bien de combler l'orifice herniaire par un bouchon de textile synthétique, fréquemment appelé "plug".

Les implants actuellement utilisés pour cette technique sont constitués de plusieurs couches ou de plusieurs feuillets de matériau polymère, tricoté ou non tissé.

Ils ont une forte densité en matériau, très localisée, et ont pour inconvénient important de délimiter des zones que les cellules immunitaires ne peuvent pas toujours parfaitement atteindre.

La présente invention a pour objet premier de pallier cet inconvénient et de proposer un implant de faible densité et de faible grammage permettant une colonisation cellulaire entre ses fibres ainsi qu'une circulation de fluides entre ses différentes faces.

L'invention a également pour objet de proposer un implant ayant une forme anatomique lui permettant de s'adapter et de s'appliquer à toutes les physionomies des sites d'implantation.

L'invention a en outre pour objet de proposer un implant particulièrement adapté à une implantation dans le cadre d'une chirurgie ouverte mini-invasive.

La plaque de réfection anatomique selon l'invention est caractérisée en ce qu'elle est constituée d'une poche de forme sensiblement pyramidale tronquée ou parallélépipédique, associée par l'une de ses faces à une collerette munie d'au moins un orifice.

Selon une autre caractéristique de la prothèse selon l'invention, le ou les orifices de la collerette sont de forme circulaire, ovoïde, carrée, rectangulaire ou triangulaire.

Selon une autre caractéristique, la prothèse comprend un orifice relié à la périphérie de la collerette par une fente.

Selon une autre caractéristique de la prothèse selon l'invention, la collerette est allongée et est en forme de "goutte d'eau", c'est-à-dire présente une extrémité arrondie et, du côté opposé, une extrémité effilée.

Selon une autre caractéristique de la prothèse selon l'invention, ledit orifice relié à la périphérie de la collerette par une fente est aménagé près de la poche, dans l'extrémité de la collerette présentant une forme arrondie.

Selon une autre caractéristique de la prothèse selon l'invention, la poche possède zéro, une ou plusieurs faces ouvertes.

Selon une autre caractéristique de la prothèse selon l'invention, la poche est reliée à la collerette indifféremment par sa base la plus grande ou par sa base la plus petite.

Selon une autre caractéristique, la prothèse est constituée d'un ou plusieurs matériaux souples et biocompatibles pouvant être biologiques, synthétiques résorbables ou synthétiques non résorbables tels que par exemple du polypropylène tricoté et / ou non tissé.

Selon une autre caractéristique, la prothèse selon l'invention est déformable permettant ainsi à ses différentes parties de s'adapter à l'anatomie ainsi qu'à la cinétique de la zone implantée.

Les éléments constitutifs de la prothèse selon l'invention sont détaillés dans la description qui suit. Cette description, purement illustrative et non limitative, doit être mise en relation avec les figures annexées et ci-après explicitées :
- La figure 1 : Vue en perspective d'une prothèse selon l'invention,
- La figure 2 : Vue du dessus de cette prothèse,
- La figure 3 : Vue de cette prothèse par le côté.

Comme le montrent les figures, la prothèse de réfection anatomique selon l'invention est constituée d'une poche 1 de forme sensiblement pyramidale tronquée ou parallélépipédique, ouverte, dans l'exemple représenté, au niveau de sa base 2. Cette poche 1 est associée, au niveau de sa base 2, à une collerette 3 allongée et en forme de "goutte d'eau", c'est-à-dire présentant une extrémité arrondie et, du côté opposé, une extrémité effilée.

Cette collerette 3 comprend une série d'orifices 4 de forme circulaire, aménagés le long de ses bords longitudinaux.

La collerette 3 peut présenter plusieurs orifices permettant la libre circulation de fluides corporels et facilitant son ancrage aux tissus environnants par suture ou collage.

Un des orifices 5 est relié à la périphérie de la collerette 3 par une fente 6 et permet le passage du cordon anatomique. Après engagement de ce cordon, les deux berges 7 et 8 de la collerette 3 sont jointes par collage, suture ou agrafage afin de repousser ou d'empêcher la survenue d'un sac herniaire.

Dans l'exemple représenté sur les dessins, l'orifice 5 est aménagé près de la poche 1, dans l'extrémité de la collerette 3 présentant une forme arrondie, et la fente 6 est aménagée selon l'axe longitudinal de la collerette 3.

Le matériau constituant cette collerette 3 permet son application aux structures tissulaires environnantes grâce à sa souplesse et permet, par découpe per-opératoire, son adaptation à la physionomie du site d'implantation.

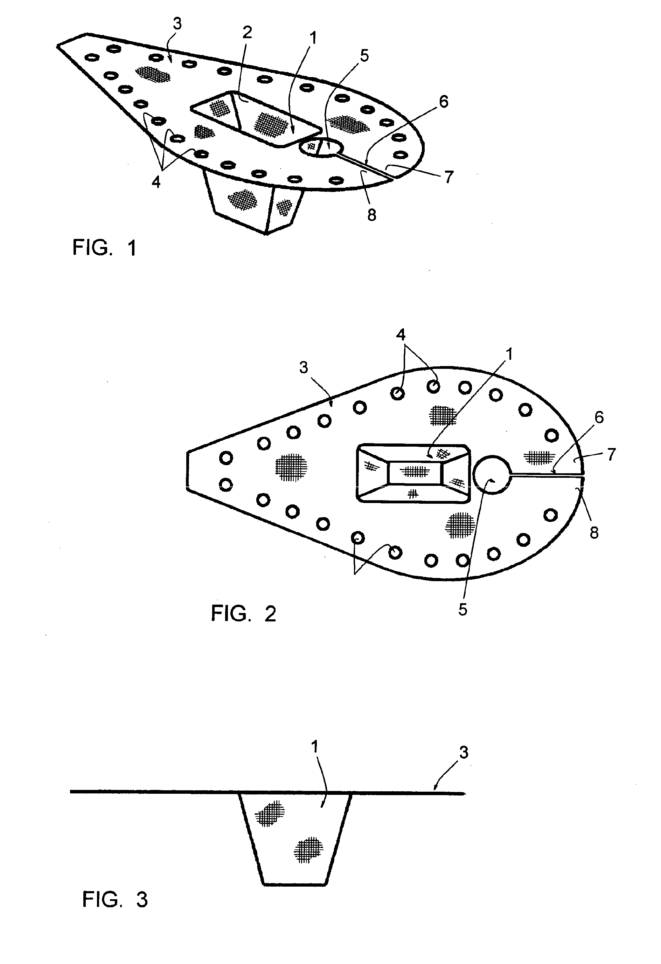

Vue de profil (figure 3), la prothèse a une forme rappelant un chapeau renversé. La poche 1 est introduite dans l'orifice à combler et repousse le sac herniaire tandis que la collerette 3 constitue un soutènement et un renfort des berges de cet orifice.

La conformation de la prothèse 1 lui confère une triple stabilité :
- une stabilité primaire par effet bouchon qu'exerce la poche 1 à l'intérieur de l'orifice herniaire : le bouchon que forme la poche repousse le sac herniaire et son contenu à l'intérieur de la cavité abdominale ;
- une stabilité secondaire par auto-blocage du fait de la configuration tri-dimensionnelle de la poche 1 ; cette stabilité est accrue par l'élasticité du matériau constituant la poche 1, qui permet à celle-ci de se refermer sous l'effet de la pression abdominale ;
- une stabilité tertiaire des différents composants de cette prothèse par ajout de sutures, agrafes, colle ou tout autre mode de fixation ;
- une stabilité quaternaire assurée par la colonisation cellulaire de la prothèse ainsi que par une fibrose cicatricielle.

Cette correction de la hernie est complétée par un renfort des parois environnantes grâce à la collerette 3 permettant ainsi de prévenir toute récidive.

La prothèse est souple et peut se déformer pour s'adapter aux contraintes physiologiques exerçant de fortes pressions dans la cavité abdominale (par exemple toux, éternuements). Cette déformation est possible suivant la largeur de la prothèse et est impossible selon sa longueur.

La prothèse est constituée de un ou plusieurs matériaux textiles biocompatibles. Ces matériaux peuvent être de type non tissé, tissé ou tricoté et constitués de fibres biologiques, synthétiques résorbables ou synthétiques non résorbables. Les matériaux utilisés peuvent être à base de polypropylène, de polyester, de polyuréthane, de polyamide, d'acide polyglycolique (PGA), d'acide poly-L-lactique (PLLA).

Dans l'application particulière du traitement des hernies inguinales et crurales la poche 1 est introduire dans l'orifice herniaire et la collerette 3 est appliquée sur les berges limitant cet orifice. La conformation allongée de la collerette 3 lui permet d'être à la fois fixée au pubis par une extrémité longitudinale et de contourner le cordon spermatique par son autre extrémité longitudinale. La collerette 3 permet la prévention d'une récidive qu'elle soit directe ou oblique externe.

La prothèse selon l'invention est ainsi différente des implants concurrents par sa forme anatomique lui permettant de s'adapter et de s'appliquer à toutes les physionomies des sites d'implantation. Sa conception ajourée de faible densité permet d'implanter dans le corps humain un corps étranger dont les caractéristiques d'encombrement sont plus favorables que les prothèses actuellement utilisées.

## Revendications

1. Plaque de réfection anatomique destinée à la réparation de hernies et particulièrement mais non exhaustivement de hernies inguinales et crurales **caractérisée en ce qu'**elle est constituée d'une poche (1) de forme sensiblement pyramidale tronquée ou parallélépipédique, et associée par l'une de ses faces (2) à une collerette (3) munie d'au moins un orifice (4).

2. Prothèse selon la revendication 1, **caractérisée en ce que** le ou les orifices (4) de la collerette (3) sont de forme circulaire, ovoïde, carrée, rectangulaire ou triangulaire.

3. Prothèse selon la revendication 1 ou la revendication 2, **caractérisée en ce qu'**elle comprend un orifice (5) relié à la périphérie de la collerette (3) par une fente (6).

4. Prothèse selon l'une quelconque des revendications précédentes, **caractérisée en ce que** la collerette (3) est allongée et est en forme de "goutte d'eau", c'est-à-dire présente une extrémité arrondie et, du côté opposé, une extrémité effilée.

5. Prothèse selon la revendication 4, **caractérisée en ce que** l'orifice (5) relié à la périphérie de la collerette (3) par une fente (6) est aménagé près de la poche (1), dans l'extrémité de la collerette (3) présentant une forme arrondie.

6. Prothèse selon l'une quelconque des revendications précédentes, **caractérisée en ce que** la poche (1) possède zéro, une ou plusieurs faces ouvertes.

7. Prothèse selon l'une quelconque des revendications précédentes, **caractérisée en ce que** la poche (1), lorsqu'elle est de forme sensiblement pyramidale tronquée, est reliée à la collerette (3) indifféremment par sa base la plus grande ou par sa base la plus petite.

8. Prothèse selon l'une quelconque des revendications précédentes, **caractérisée en ce qu'**elle est constituée d'un ou plusieurs matériaux souples et biocompatibles pouvant être biologiques, synthétiques résorbables ou synthétiques non résorbables tels que du polypropylène tricoté et / ou non tissé.
